# FASCICULE DE BREVET EUROPEEN

(11) **EP 3 283 799 B1**
(45) Date de publication et mention de la délivrance du brevet: **05.06.2024**
(21) Numéro de dépôt: 16716606.5
(22) Date de dépôt: 15.04.2016
(51) Int. Cl.: F16K 11/085, F16K 27/06, A61M 39/10, A61M 39/22

(54) **ROBINET MEDICAL, KIT COMPRENANT UN TEL ROBINET ET METHODE DE PREPARATION D'UN MELANGE OU D'UNE EMULSION**
MEDIZINISCHES VENTIL, KIT MIT SOLCH EINEM VENTIL UND VERFAHREN ZUR HERSTELLUNG EINES GEMISCHES ODER EINER EMULSION
MEDICAL VALVE, KIT COMPRISING SUCH A VALVE, AND METHOD FOR PREPARING A MIXTURE OR AN EMULSION

(30) Priorité: 15.04.2015 FR 1553326
(43) Date de publication de la demande: 21.02.2018
(73) Titulaire: Guerbet, 93420 Villepinte (FR)
(72) Inventeur: ALLARD, Ludovic, 69390 Millery (FR)
(74) Mandataire: Lavoix
(86) Numéro de dépôt international: PCT/EP2016/058441
(87) Numéro de publication internationale: WO 2016/166339

(56) Documents cités:
- EP-A1- 1 839 699
- EP-A1- 2 078 536
- WO-A1-2012/024370
- WO-A1-2014/090958
- BE-A1- 873 375
- DE-U1-202013 103 615
- US-A- 4 807 666
- US-A1- 2009 299 270
- US-A1- 2012 245 564
- US-A1- 2013 030 348
- US-A1- 2014 276 215

## Description

L'invention concerne un robinet médical, un kit de préparation d'un produit à injecter, préférentiellement un mélange ou une émulsion, comprenant un tel robinet médical, et une méthode de préparation d'un produit ou d'une émulsion destinée à l'injection chez un patient mise en oeuvre à l'aide d'un tel kit de préparation.

Depuis près de trente ans, les huiles iodées sont utilisées dans des procédures de radiologie interventionnelle. Le Lipiodol^{®} se caractérise par sa propension à être capté sélectivement par les tumeurs hépatiques. Cette huile est donc utilisée comme vecteur d'agent anticancéreux pour le traitement du carcinome hépatocellulaire dans une technique appelée « chimio-embolisation intra-artérielle » ou « TransArterial ChemoEmbolisation » (TACE) en anglais (Nakamura et al.: Radiology, 1989 ; 170:783-6 et J.M. Idée - B. Guiu : Critical Reviews in Oncology/Hematology, 2013 ; 88(3):530-49). Les huiles iodées et en particulier le Lipiodol^{®} sont aussi connues pour induire une embolisation transitoire de la circulation artérielle provoquant ainsi un ralentissement de celle-ci. Etant donné que la plupart des agents anticancéreux sont solubles dans l'eau, la forme « émulsion », qui est adaptée pour le mélange de deux phases non solubles l'une dans l'autre, apparait comme étant la plus judicieuse pour mélanger une huile iodée et un agent anticancéreux. Elle semble être la plus adaptée pour transporter et délivrer dans une tumeur un agent anticancéreux, trop toxique et pas assez efficace quand il est administré non émulsifié par voie intra-artérielle ou par voie systémique.

Pour réaliser une TACE, un radiologue interventionnel prépare extemporanément l'émulsion, juste avant l'injection. Il utilise classiquement deux seringues de 50 mL connectées à un robinet 3 ports pourvu de deux connecteurs femelles auxquels sont connectées les seringues et d'un connecteur mâle auquel un cathéter ou un micro-cathéter peut être fixé. L'une des seringues contient une solution d'un agent anticancéreux tandis que l'autre seringue contient une huile iodée comme le Lipiodol^{®}. L'émulsion est obtenue par exemple après dix passages successifs rapides du contenu d'une seringue à l'autre, à l'aide d'un robinet trois voies. L'émulsion est alors transférée dans l'une des deux seringues de mélange, la seringue de mélange vide est déconnectée du robinet et une seringue d'injection est alors mise à la place de la seringue de mélange qui a été enlevée. Une petite quantité d'émulsion est transférée dans la seringue d'injection en actionnant le piston de la seringue de mélange restante. L'injection est réalisée en dix minutes dans la branche droite ou gauche de l'artère hépatique d'un patient irriguant la plus grande partie de la tumeur.

Etant donné qu'il y a parfois plusieurs injections successives qui sont réalisées, l'émulsion restante dans la seringue de mélange est parfois mélangée à nouveau comme décrit ci-dessus, après déconnexion de la seringue d'injection et reconnexion de la seconde seringue de mélange. Une nouvelle injection est réalisée après le transfert de l'émulsion dans l'une des deux seringues de mélange, la déconnexion de la seringue de mélange vide du robinet, la connexion d'une seringue d'injection à la place de la seringue de mélange qui a été enlevée et le transfert d'une petite quantité d'émulsion dans la seringue d'injection en actionnant le piston de la seringue de mélange restante.

Les robinets en matériau plastique actuellement sur le marché qui sont destinés à un usage médical et en particulier qui sont destinés à réaliser la préparation d'une émulsion qui sera injectée à un patient dans le cadre d'une chimio-embolisation intraartérielle, sont majoritairement des robinets trois ports et présentent différents problèmes gênants pour le radiologue interventionnel pratiquant cette technique :
- ces robinets sont rarement directement connectés à un cathéter ou à un micro-cathéter. La seringue d'injection est le plus souvent déconnectée du robinet puis connectée directement à l'un de ces dispositifs avals.
- ces robinets impliquent de déconnecter une ou plusieurs fois une seringue de mélange et la seringue d'injection. Cela augmente les risques de contamination microbienne et de mise en contact des praticiens avec les produits contenus dans ces seringues.
- ces robinets n'indiquent le plus souvent pas clairement la voie qui est ouverte.
- ces robinets ne présentent pas de dispositif permettant d'éviter la connexion d'une seringue de mélange sur le port destiné à la seringue d'injection.
- ces robinets ne présentent pas le plus souvent de dispositif facilitant sa préhension et notamment la connexion ou la déconnexion des seringues.

Le brevet FR 2 804 609 décrit un robinet à quatre ports qui inclut des moyens de connexion reconfigurables manuellement pour permettre l'administration d'un produit de contraste via différentes tubulures à un patient. Trois ports du robinet comportent à leur extrémité un filetage permettant la liaison d'un cône Luer, et le quatrième port est adapté pour la liaison d'un cathéter par l'intermédiaire d'un connecteur rotatif. Un tel robinet médical ne permet cependant pas d'éviter la connexion d'une seringue de mélange sur le port destiné à la seringue d'injection.

DE 20 2013 103 615 décrit un robinet médical comportant des ports munis d'éléments protubérants permettant de provoquer des fuites lorsque des seringues munies de connecteurs incompatibles sont branchées, afin que le praticien s'aperçoive de l'erreur de branchement. Une telle solution ne permet pas d'empêcher les erreurs de manipulation de façon suffisamment fiable.

EP 2 078 536 divulgue un robinet médical comprenant un corps muni de trois connecteurs femelles et d'un connecteur mâle. Deux des connecteurs femelles sont prévus pour respectivement recevoir des première et deuxième seringues contenant respectivement un premier et un second liquide, tandis que le troisième connecteur femelle est prévu pour recevoir une troisième seringue dans laquelle les premier et second liquides sont envoyés depuis les première et deuxième seringues via l'intérieur du robinet, avant d'être mélangés dans cette troisième seringue. Ce robinet ne permet pas d'éviter de monter la troisième seringue sur les connecteurs femelles prévus pour les première et deuxième seringues.

C'est à cet inconvénient qu'entend remédier l'invention en proposant un nouveau robinet médical permettant d'éviter la connexion d'une seringue de mélange sur le port destiné à la seringue d'injection et les inconvénients qui en découlent.

A cet effet, l'invention concerne un robinet médical, tel que défini à la revendication 1.

Grâce à l'invention, ce robinet médical permet, quand on lui connecte des seringues de différents produits, de réaliser un mélange de solution injectable. Cela permet d'obtenir un mélange ou une émulsion d'une solution aqueuse, de préférence d'un agent anti-cancéreux et d'une huile iodée, et de transférer ce mélange ou cette émulsion dans une seringue d'injection connectée sur un connecteur de ce robinet qui lui sera spécifiquement dédié grâce au détrompeur. Les risques d'erreurs de manipulation sont donc réduits. On considère que l'invention concerne un ensemble médical comprenant un robinet tel que mentionné ci-dessus, au moins une seringue de mélange et au moins une seringue d'injection.

Selon des aspects avantageux mais non obligatoires de l'invention, un tel robinet médical peut incorporer une ou plusieurs des caractéristiques énoncées aux revendications 2 à 9.

L'invention concerne également un kit de préparation d'un produit à injecter, tel que défini à la revendication 10.

Selon des aspects avantageux mais non obligatoires de l'invention, un tel kit de préparation peut incorporer une ou plusieurs des caractéristiques énoncées aux revendications 11 et 12.

L'invention concerne également une méthode de préparation d'un mélange ou d'une émulsion destiné à l'injection chez un patient, telle que définie à la revendication 13.

Un aspect avantageux mais non obligatoire de la méthode de l'invention est spécifié à la revendication 14.

L'invention sera mieux comprise et d'autres avantages de celle-ci apparaitront plus clairement à la lumière de la description qui va suivre d'un robinet médical, d'un kit de préparation d'un produit, préférentiellement un mélange ou une émulsion et d'une méthode de préparation d'un produit, préférentiellement un mélange ou une émulsion, conformes à son principe, faite à titre d'exemple non limitatif en référence aux dessins annexés dans lesquels :
- la figure 1 est une vue en perspective d'un robinet médical conforme à l'invention ;
- la figure 2 est une vue de dessous du robinet médical de la figure 1 ;
- la figure 3 est une coupe du robinet médical des figures 1 et 2, selon un plan comprenant les axes centraux de quatre connecteurs du robinet médical;
- la figure 4 est une vue en perspective d'un boisseau du robinet médical des figures 1 à3 ;
- la figure 5 est une vue en perspective, selon un autre angle, du boisseau de la figure 4 ;
- la figure 6 est une vue en perspective, selon un autre angle, d'un autre mode de réalisation d'un boisseau;
- la figure 7 est une vue en perspective du robinet médical de la figure 1, dont le boisseau est omis ;
- la figure 8 est une coupe du robinet médical des figures 1 et 2, selon un plan passant par la base d'un levier du boisseau ;
- la figure 9 est une vue en perspective du robinet médical des figures 1 à 3, d'un cathéter connecté sur un connecteur du robinet médical et une seringue de mélange positionnée devant un connecteur femelle du robinet médical;
- la figure 10 est une vue en perspective de l'extrémité d'une seringue de mélange ;
- la figure 11 est une vue en perspective d'une partie d'un kit de préparation conforme à l'invention.

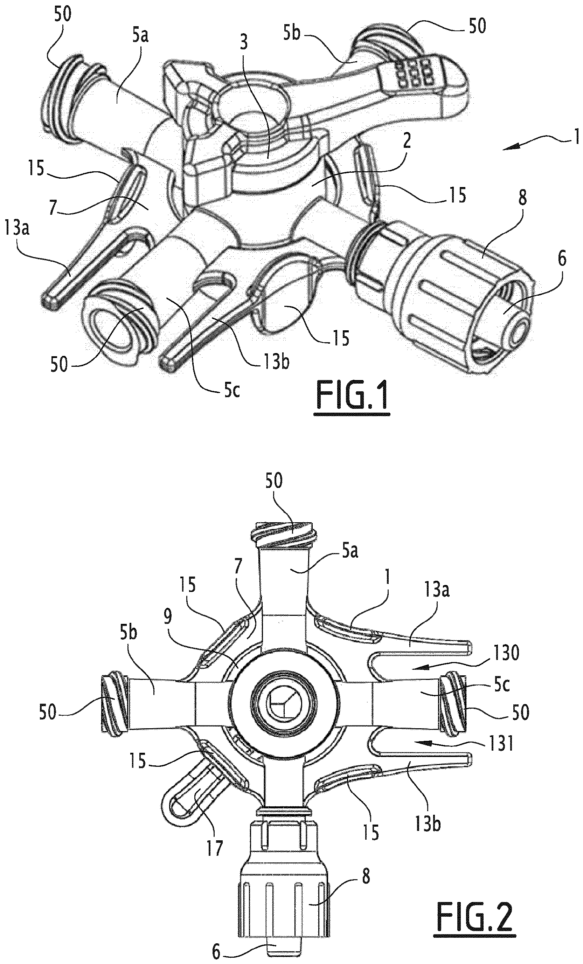

Les figures 1 à 3 représentent un robinet médical 1 permettant de préparer un mélange, préférentiellement une émulsion, destiné à être injecté à un patient. Le robinet médical 1 comprend un corps 2 creux à partir duquel s'étendent trois connecteurs femelles 5a, 5b et 5c et un connecteur mâle 6, préférentiellement un connecteur luer. Le corps 2 comprend un alésage central 200 à partir duquel s'étendent quatre conduits 2a, 2b, 2c et 2d parcourant respectivement les connecteurs 5a, 5b, 5c et 6.

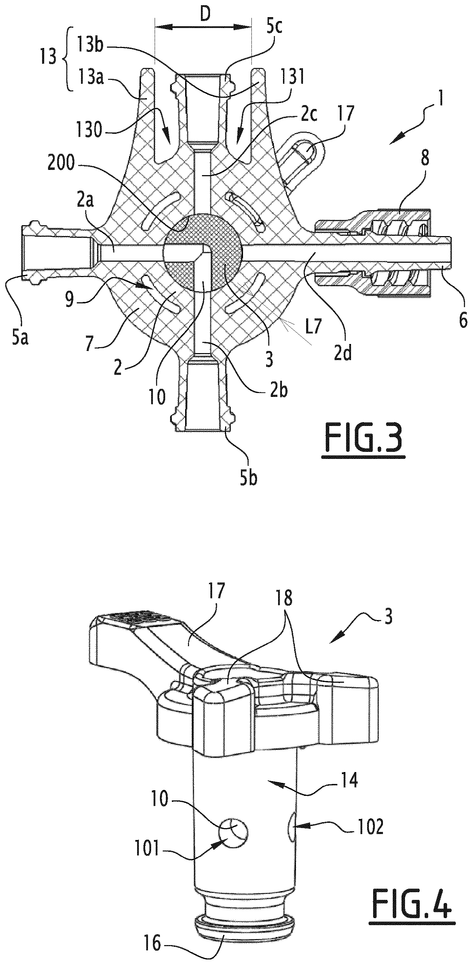

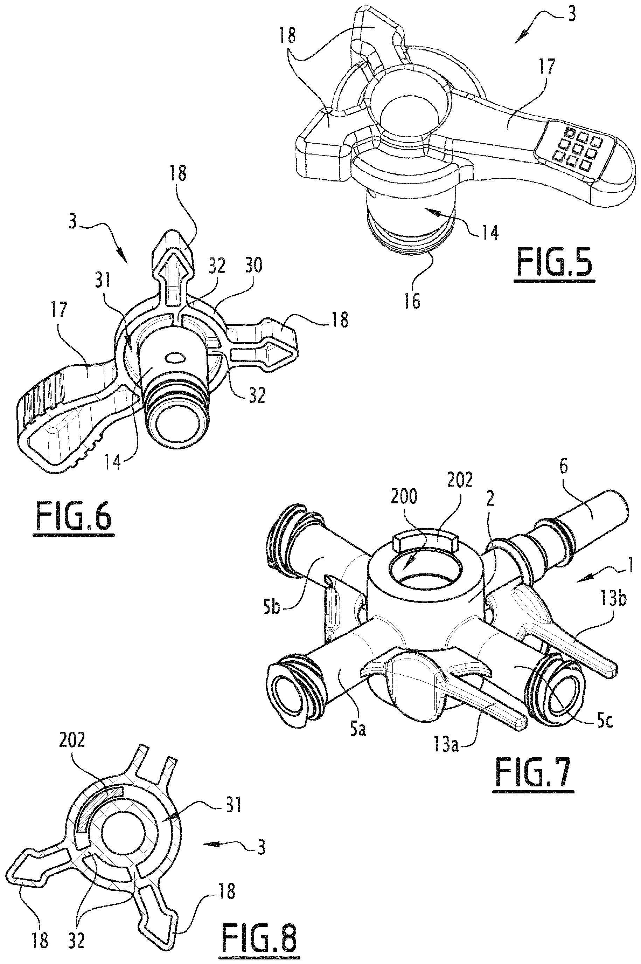

Le robinet 1 comprend un boisseau 3 représenté notamment sur les figures 4 et 5, monté rotatif dans l'alésage 200. Le boisseau 3 comprend un canal de circulation de fluide 10 permettant de mettre en communication les conduits 2a, 2b, 2c et 2d selon un mode décrit ci-après. Le boisseau 3 comprend une paroi cylindrique externe 14 permettant de réaliser l'étanchéité du robinet 1 avec l'alésage 200. Une extrémité axiale du boisseau 3 comprend une collerette 16 permettant son maintien dans le corps 2 par clippage.

Il existe d'autres solutions de maintien du boisseau 3 dans le corps 2, telles que le bouterollage ou l'ajout d'une pièce de maintien. Par « bouterollage », on entend, au sens de la présente invention, la création d'une liaison mécanique entre le boisseau 3 et le corps 2 entrainant la déformation partielle du boisseau 3.

Le boisseau 3 comprend de préférence un levier 17 actionnable par un utilisateur permettant la rotation du boisseau 3 autour de son axe central.

Conformément à l'invention, l'un des connecteurs femelles, le connecteur 5c dans l'exemple représenté, comprend un détrompeur 13. Par « détrompeur », on entend désigner un dispositif mécanique permettant d'éviter les erreurs d'assemblage, de montage ou de branchement en fournissant une indication visuelle et en définissant une configuration mécanique qui empêche l'utilisation d'éléments non désirés. Le détrompeur 13 comprend deux cornes 13a et 13b prévues de part et d'autre du connecteur femelle 5c et qui définissent deux espaces d'insertion 130 et 131 situés respectivement entre les cornes 13a et 13b et le connecteur 5c.

Le détrompeur 13 permet préférentiellement d'empêcher la connexion d'une seringue dont le volume est supérieur à une valeur prédéterminée, grâce à la géométrie des cornes 13a et 13b et la largeur des espaces 130 et 131 ou d'une seringue pourvue d'un moyen rendant incompatible sa connexion avec le connecteur 5c comprenant ce détrompeur 13 et/ou ces cornes 13a et 13b du fait de l'incompatibilité entre ce moyen et la configuration mécanique définie par le détrompeur 13 et/ou les cornes 13a et 13b de ce détrompeur 13. Encore plus préférentiellement, le détrompeur 13 et de manière avantageuse, les cornes 13a et 13b de ce détrompeur 13, permettent d'empêcher le montage de seringues 20 et 21, préférentiellement des seringues de mélange, pourvues d'un moyen rendant incompatible leur connexion avec le connecteur 5c comprenant ce détrompeur 13 et/ou les cornes 13a et 13b de ce détrompeur. Préférentiellement, quand les cornes 13a et 13b empêchent la connexion d'une seringue pourvue d'un moyen rendant incompatible sa connexion avec le connecteur femelle 5c comprenant ce détrompeur 13 et/ou ces cornes 13a et 13b du fait de l'incompatibilité entre ce moyen et la configuration mécanique définie par le détrompeur et/ou les cornes de ce détrompeur, elles ne dépassent pas de plus de 5 mm le bout du connecteur femelle 5c. De préférence, quand les cornes 13a et 13b empêchent la connexion d'une seringue dont le réservoir a un diamètre supérieur à une valeur seuil correspondant à un volume maximal, préférentiellement d'une seringue de mélange, elles dépassent de plus de 5 mm le bout du connecteur femelle.

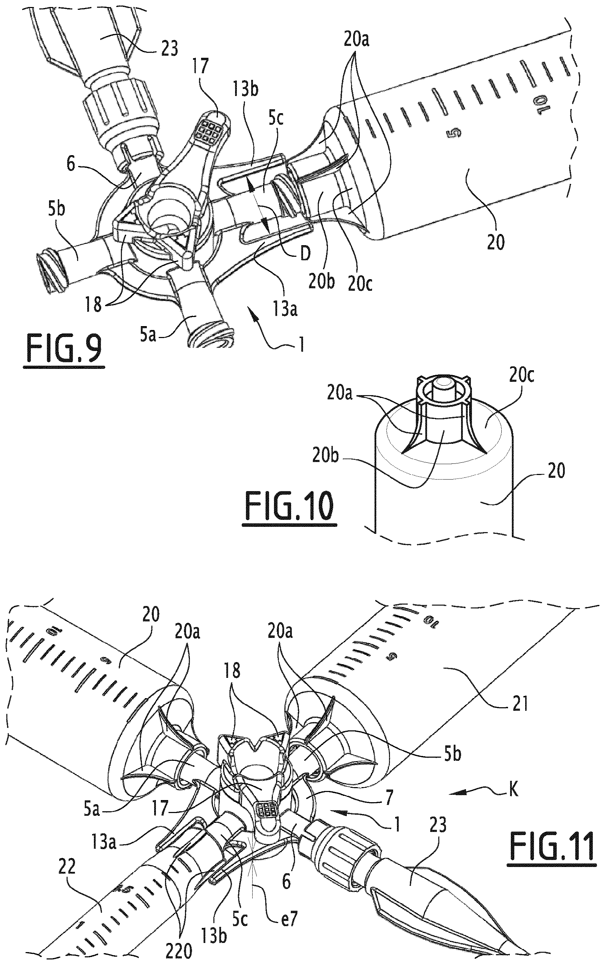

Dans l'exemple représenté à la figure 9, la seringue de mélange 20 est pourvue de moyens rendant incompatible sa connexion avec le connecteur 5c comprenant le détrompeur 13. Dans ce mode de réalisation, la seringue de mélange 20 comprend à son extrémité des ailettes 20a, formant les moyens rendant incompatible la connexion de la seringue de mélange 20 avec le connecteur 5c comprenant un détrompeur. Pour effectuer le vissage d'une seringue de mélange sur un connecteur femelle, préférentiellement du type luer, il faut réaliser entre un et deux tours en fonction du luer. Ainsi, la seringue de mélange 20 comprend au moins une ailette 20a, préférentiellement au moins deux ailettes 20a. Cela permet que le détrompeur 13 assure sa fonction. L'extrémité de la seringue de mélange 20 est formée par un connecteur luer comprenant un filetage mâle interne prévu sur une jupe périphérique 20b. Les ailettes 20a sont prévues sur la surface externe de la jupe 20b. Comme montré sur la figure 9, les ailettes 20a créent un encombrement à l'extrémité de la seringue de mélange 20, autour de la jupe 20b du filet mâle du connecteur luer, et qui est supérieur à la distance D entre les cornes 13a et 13b. De manière plus préférentielle, la seringue de mélange 20 comprend à son extrémité quatre ailettes 20a positionnées à 90° les unes par rapport aux autres. Cela permet d'assurer un meilleur équilibre dimensionnel du corps de la seringue ainsi qu'une meilleure ergonomie en termes de préhension de celle-ci. De manière préférentielle, les ailettes 20a de la seringue de mélange 20 ont une forme qui permet de générer une interférence avec les cornes 13a et 13b du détrompeur 13. De manière avantageuse, ces ailettes 20a permettent de faciliter le vissage de la seringue de mélange 20 sur les connecteurs 5a ou 5b, en améliorant la préhension de celle-ci. De manière avantageuse, les ailettes sont contigües à la jupe externe 20b du filet « luer lock » mâle ainsi qu'à une zone conique terminale 20c du réservoir de la seringue. De manière préférentielle, ces ailettes 20a ont une forme externe en arc de cercle. Cela permet de positionner les doigts sur ces ailettes et d'assurer une bonne préhension de la seringue de mélange 20 tout en restant atraumatique.

La seringue de mélange 21 est également pourvue d'une telle géométrie.

Les connecteurs 5a, 5b et 5c comprennent un filetage 50, sur lequel un moyen de connexion permettant l'assemblage d'un dispositif médical de manière étanche se connecte. De manière préférentielle, ce moyen de connexion est un luer qui est le moyen de connexion standard de référence dans le domaine médical et qui se visse sur le filetage 50. Ce moyen de connexion se raccorde au réservoir de la seringue. Les cornes 13a et 13b permettent de dimensionner les espaces d'insertion 130 et 131 de manière qu'il soit impossible d'y introduire des seringues dont le réservoir a un diamètre supérieur à une valeur seuil correspondant à un volume maximal ou qu'il soit impossible de connecter une seringue du fait de l'incompatibilité entre au moins un moyen présent sur ladite seringue et la configuration mécanique définie par le détrompeur et/ou ses cornes 13a et 13b.

De préférence, le détrompeur 13 permet d'empêcher le montage sur le connecteur 5c de seringues présentant un moyen rendant incompatible leur connexion avec la configuration mécanique définie par le détrompeur ou avec des seringues dont le volume est supérieur à 3 ml. De manière plus préférentielle, le détrompeur 13 permet d'empêcher le montage sur le connecteur 5c de seringues présentant au moins un moyen rendant incompatible leur connexion avec la configuration mécanique définie par le détrompeur. La présence d'ailettes à l'extrémité des seringues de mélange 20 et 21 rend leur connexion incompatible avec la configuration mécanique définie par le détrompeur comme cela est montré sur la figure 9. Ainsi, il n'est pas possible dans ce mode de réalisation, de connecter au connecteur femelle 5c, une seringue de mélange.

Le risque d'erreur de manipulations faisant courir le risque aux patients est fortement réduit car en empêchant la connexion avec une seringue de mélange, la connexion fluidique est impossible du fait des moyens rendant incompatible la connexion, formés sur la seringue de mélange par les ailettes 20a. Il n'y a donc pas de risque qu'une substance erronée soit injectée au patient, car tout transfert de fluide est impossible, la seringue de mélange ne pouvant être solidarisée mécaniquement au connecteur du robinet, au contraire du système décrit dans DE 20 2013 103 615.

Le connecteur femelle 5c est préférentiellement adapté à être connecté avec une seringue d'injection 22, comme cela est représenté à la figure 11. Le diamètre extérieur de cette seringue 22 et/ou l'encombrement de l'extrémité de cette seringue 22 lui permettent d'être insérée dans les espaces d'insertion 130 et 131 lors de son vissage sur le connecteur 5c. De manière préférentielle, la seringue d'injection 22 comprend à son extrémité distale des godrons de préhension 220 qui ont un encombrement inférieur à la distance D entre les cornes 13a et 13b.

Dans un autre mode de réalisation, une seringue de volume supérieur à 3 ml, comme une seringue de mélange 20, ne pourra pas être montée sur le connecteur 5c à cause du diamètre trop important de son réservoir.

Les connecteurs 5a et 5b ne comportent pas de détrompeur 13 et sont donc adaptés pour le montage de seringues de mélange, comme cela est représenté à la figure 11, sur laquelle deux seringues de mélange 20 et 21 sont montées sur les connecteurs 5a et 5b.

Par « seringue d'injection », on entend préférentiellement désigner une seringue de faible volume, c'est-à-dire de volume compris entre 1 ml et 3 ml. Par « seringue de mélange », on entend préférentiellement désigner une seringue de grand volume, c'est-à-dire une seringue d'un volume supérieur ou égal à 10 ml.

De façon avantageuse, les connecteurs femelles 5a et 5b sont contigus, ce qui permet la connexion de deux seringues de mélange contiguës.

De manière avantageuse, le canal de circulation de fluide 10 met en communication uniquement deux des connecteurs 5a, 5b, 5c et 6 du robinet 1. A cet effet, le canal de circulation de fluide 10 ne comprend qu'une seule voie munie de deux ouvertures 101 et 102 sur la paroi cylindrique externe 14. L'utilisation du robinet médical 1 est ainsi sécurisée. En effet, seulement deux voies sont en communication quelle que soit l'orientation du boisseau 3, ce qui permet de réaliser trois actions mais uniquement une de ces trois actions à la fois :
- le mélange d'une première substance et d'une seconde substance contenues respectivement dans les seringues de mélange 20 et 21, connectées sur les connecteurs 5a et 5b, ou
- le transfert d'une partie du mélange obtenu et contenu dans l'une des seringues de mélange 20 et 21 dans la seringue d'injection 22 connectée sur le connecteur 5c, ou
- l'injection chez un patient, au travers d'un cathéter connecté sur le connecteur 6, du mélange préalablement transféré dans la seringue d'injection 22 connectée sur le connecteur 5c.

Ainsi, il ne sera pas possible d'injecter le mélange chez un patient tout en effectuant le remplissage de la seringue d'injection 22 ou d'effectuer le mélange de la première et de la seconde substance contenues dans les seringues de mélange 20 et 21 tout en transférant le mélange obtenu dans la seringue d'injection 22. De manière préférentielle, le mélange est une émulsion.

A cet effet, le canal de circulation de fluide 10 est en forme de L, ce qui permet de mettre en communication fluidique les seringues de mélange 20 et 21, ou la seringue de mélange 20 avec la seringue d'injection 22 ou la seringue d'injection 22 avec un dispositif aval tel qu'un cathéter 23 ou un micro-cathéter.

De manière avantageuse, le boisseau 3 comprend des indicateurs visuels 18 permettant de signaler à l'utilisateur du robinet 1 quels sont les connecteurs qui sont en communication par le positionnement du boisseau 3.

Dans l'exemple représenté, les indicateurs 18 sont deux flèches orientées à 90° l'une par rapport à l'autre permettant de montrer le positionnement du canal 10 de circulation de fluide.

De manière encore plus avantageuse, comme cela est représenté aux figures 6 à 8, le boisseau 3 comprend, sur le dessous d'une partie supérieure 30 qui porte le levier 17 et les indicateurs 18, une rainure circulaire 31 interrompue par des nervures 32 alignées avec les indicateurs visuels 18. Le corps 2 du robinet médical comprend sur une partie supérieure un moyen empêchant une rotation du boisseau 3 qui mettrait en communication le connecteur auquel une seringue de mélange est connectée et le connecteur auquel est connecté un dispositif aval (c'est-à-dire qui mettrait en communication les connecteurs 5b et 6). Ce moyen est formé par une nervure 202 de forme courbe et adaptée pour coulisser dans la rainure circulaire 31 entre les nervures 32.

Ce robinet 1 permet ainsi soit d'effectuer un mélange ou une émulsion de deux substances contenues dans des seringues de mélange 20 et 21, soit de remplir une seringue d'injection 22 à partir de l'une des seringues de mélange (préférentiellement la seringue 20), soit d'injecter le mélange ou l'émulsion obtenu à un patient. Le dispositif selon l'invention est donc préférentiellement un robinet 3 voies, 4 ports.

La position des nervures 32 est préférentiellement liée à la position de la rainure circulaire 31 sur le boisseau 3. Les nervures 32 pourraient donc, dans un autre mode de réalisation, être positionnées différemment, c'est-à-dire qu'elles pourraient ne pas être alignées avec les indicateurs visuels 18, à la condition que leur positionnement permette d'empêcher la mise en communication des conduits 2b et 2d.

Avantageusement, le robinet médical 1 comprend au moins deux zones de préhension adaptées au positionnement d'un doigt. Ces zones de préhension permettent de saisir plus efficacement le robinet 1 et de faciliter ainsi sa manipulation lors des étapes de connexion ou de déconnexion des seringues 20, 21 et 22 ou lors de l'orientation du boisseau 3 permettant la mise en communication de tel ou tel connecteur.

Dans l'exemple représenté, le robinet 1 comprend quatre zones de préhension 15 formées par des formes circulaires planes faisant saillie du corps 2. Ces zones de préhension 15 sont réparties entre les connecteurs 5a, 5b, 5c et 6.

De façon optionnelle, le robinet médical 1 comprend en outre une collerette de renfort 7 solidaire d'au moins deux des connecteurs du robinet médical 1 et espacée du corps central 2 pour former une zone d'ajourage 9.

Avantageusement, la collerette de renfort 7 est solidaire de trois ou quatre connecteurs du robinet médical 1. Dans l'exemple représenté, la collerette de renfort 7 est solidaire des quatre connecteurs 5a, 5b, 5c et 6 et forme une ceinture complète autour du corps 2, améliorant ainsi la solidité du robinet 1.

Il est important que l'intersection entre la collerette de renfort 7 et chaque connecteur soit éloignée du filetage 50 du connecteur, ou plus généralement de la zone fonctionnelle du connecteur, qui est le plus souvent l'extrémité distale de ce connecteur, puisque la zone fonctionnelle est le port de connexion sur lequel ou dans lequel s'insère par exemple une seringue ou la bague de verrouillage d'une tubulure ou d'un cathéter.

La collerette de renfort 7 est réalisée dans le même matériau que le corps 2. Dans ce cas, le robinet médical 1 est formé en une pièce et dans la même matière.

En variante, la collerette de renfort 7 est réalisée dans un matériau différent du corps 2 et du boisseau 3. Dans un tel cas, le robinet médical 1 est formé en une pièce, mais implique une opération de surmoulage de la collerette 7 ce qui présente l'avantage de renforcer la tenue mécanique de cette dernière. Le surmoulage de la collerette de renfort 7 est réalisé en des matériaux plastiques « chargés ». On entend par « matériau plastique chargé », un matériau dans lequel une substance solide, non miscible, appelée « charge » a été dispersée au moment de l'injection. Préférentiellement, la charge est choisie parmi les composés de la liste suivante : les charges minérales sous forme de poudre telles que de la silice synthétique, les charges organiques telles que de la farine de bois ou d'écorce de fruits ou de la pâte de cellulose, les charges renforçantes fibreuses telles que les fibres de verre et les charges renforçantes non fibreuses telles que des microsphères de verre creuses ou de la silice synthétique. Les charges renforçantes fibreuses permettent d'améliorer les caractéristiques mécaniques, la tenue thermique et la stabilité dimensionnelle du matériau. Préférentiellement, la collerette de renfort 7 est surmoulée sur le corps 2 avec une matière chargée en fibres, telles que des fibres de verre.

La collerette de renfort 7 a une largeur L7 supérieure à son épaisseur e7, ce qui permet ainsi d'améliorer le moment d'inertie et donc la tenue mécanique du robinet 1. La collerette de renfort 7 présente de préférence une largeur L7 3 à 10 fois, préférentiellement 3 à 7 fois, plus préférentiellement 3 à 5 fois plus importante que son épaisseur e7.

Préférentiellement, les moyens de préhension 15 sont situés sur la collerette de renfort 7.

Dans l'exemple représenté, les cornes 13a et 13b du détrompeur 13 s'étendent à partir de la collerette de renfort 7. En variante non représentée, les cornes 13a et 13b peuvent ne pas être connectées à la collerette de renfort 7 et s'étendre, par exemple à partir du connecteur 5c ou du corps 2.

Le corps 2, le boisseau 3 et/ou la collerette de renfort 7 qui composent le robinet médical 1 sont réalisés à partir d'un ou plusieurs matériaux qui supportent mieux les contraintes mécaniques et chimiques. Préférentiellement, le corps 2 et la collerette de renfort 7 sont dans un matériau différent du boisseau 3. Les contraintes mécaniques sont essentiellement la déformation par cisaillement et la pression exercée sur le robinet 1 lors de sa fabrication et/ou de son utilisation. De plus, le matériau de fabrication du robinet 1 doit être résistant à tout produit pharmaceutique, y compris à des produits huileux. De préférence, le matériau doit être résistant à Lipiodol^{®}. En outre ou alternativement, le matériau du corps 2 et/ou de la collerette de renfort 7 doit être caractérisé par un module de résistance mécanique (Module d'Young) élevé. Ainsi, le robinet médical 1, préférentiellement son corps 2 et sa collerette de renfort 7, est réalisé à partir des matériaux choisis dans la liste suivante : acrylonitrile butadiène styrène (ABS), copolymère méthyméthacrylate-acrylonitrile-butadiène styrène (MABS), polyester, polycarbonates (PC), alliages de polycarbonates, polysulfones, polyuréthannes, polyéthercétonecétone (PEKK), polyétheréthercétone (PEEK), polyaryléthercétones (PAEK), polymétacrylate de méthyle (PMMA), polyétherimides, polyamides (PA), préférentiellement les PA11 et PA12, polyméthylpentène (TPX), polysulfone (PSU), copolymères de cyclooléfines (COC), polymères de cyclooléfines (COP), fluoroplastiques autres que le polytétrafluoroéthylène (PTFE) et le phosphoénolpyruvate (PEP) et des combinaisons de ces matériaux (par exemple, ABS-PC). Préférentiellement, le robinet médical 1, plus préférentiellement son corps 2 et sa collerette de renfort 7, est réalisé à partir des matériaux choisis dans la liste suivante : acrylonitrile butadiène styrène (ABS), copolymère méthyméthacrylate-acrylonitrile-butadiène styrène (MABS), polycarbonates (PC), polyétheréthercétone (PEEK), polymétacrylate de méthyle (PMMA), polyamides (PA), préférentiellement les PA11 et PA12, polyméthylpentène (TPX), polysulfone (PSU), copolymères de cyclooléfines (COC), polymères de cyclooléfines (COP). De manière encore plus préférentielle, le robinet médical 1, plus préférentiellement son corps 2 et sa collerette de renfort 7, est réalisé à partir des matériaux choisis dans la liste suivante : polyétheréthercétone (PEEK), polymétacrylate de méthyle (PMMA), polyamides (PA), préférentiellement les PA11 et PA12, polyméthylpentène (TPX) et polysulfone (PSU). De préférence, le robinet médical 1 comprend du polyamide ou est constitué de polyamide.

Préférentiellement, le boisseau 3 du robinet est réalisé dans un matériau plus tendre que le corps 2 et/ou que la collerette de renfort 7 de ce robinet. Ainsi le boisseau 3 du robinet est préférentiellement réalisé à partir des matériaux choisis dans la liste suivante : polyéthylène (PE), polypropylène (PP), polyoxyméthylène (POM) ou polytéréphtalate de butylène (PBT). Ce matériau doit permettre au boisseau 3 de pouvoir légèrement se conformer dans le corps 2 du robinet. De plus, le fait que le corps 2 et que le boisseau 3 soient en des matériaux différents permet d'améliorer les propriétés de rotation du boisseau 3 dans le corps 2 du robinet. Le boisseau 3 est plus préférentiellement réalisé en POM.

Avantageusement, les connecteurs femelles 5a, 5b et 5c sont des ports d'entrée, alors que le connecteur mâle 6 est un port de sortie.

Dans un mode de réalisation, le connecteur mâle 6 comprend une bague de verrouillage 8 qui est fixe. Cette bague est donc solidaire du connecteur 6 et de façon plus générale du corps 2 du robinet 1.

Dans l'exemple représenté et de manière préférentielle, le connecteur mâle 6 comprend une bague de verrouillage 8 qui est montée par clipsage sur l'extrémité distale du connecteur mâle 6. La bague de verrouillage 8 est alors mobile et permet de renforcer la connexion du connecteur mâle 6 avec un connecteur femelle d'un dispositif aval (par exemple un cathéter 23 ou un micro-cathéter). De façon plus préférentielle, la bague de verrouillage 8 tourne sur l'axe du connecteur mâle 6 qui, lui, reste fixe. Cela a comme avantage de rendre la manipulation de connexion plus aisée sans risque de déconnexion.

Comme cela est représenté à la figure 11, le robinet médical 1 permet de former un kit de préparation K d'un produit à injecter, préférentiellement un mélange ou une émulsion, avec deux seringues de mélange 20 et 21 et une seringue d'injection 22. Les connecteurs femelles 5a et 5b sont respectivement connectés à une première seringue de mélange 20 et à une seconde seringue de mélange 21, et le connecteur femelle 5c comprenant le détrompeur 13 est connecté à une seringue d'injection 22.

Le kit de préparation peut également comprendre un dispositif aval, tel qu'un cathéter 23 ou un micro-cathéter permettant l'injection chez un patient du produit à injecter contenu dans la seringue d'injection 22. Le cathéter 23 est monté et verrouillé sur le connecteur mâle 6 grâce à la bague de verrouillage 8.

De manière préférentielle, c'est le connecteur 5c qui comprend un détrompeur 13, qui est sur la voie en communication avec un dispositif aval tel qu'un cathéter 23 ou un micro-cathéter. Préférentiellement, le détrompeur 13, selon la présente invention, empêche la connexion d'au moins un des éléments du kit, encore plus préférentiellement d'au moins deux des éléments du kit. Encore plus préférentiellement, le détrompeur 13, selon la présente invention, empêche la connexion des seringues de mélange 20, 21.

De manière préférentielle, les éléments du kit dont la connexion est empêchée par le détrompeur 13, sont pourvus de moyens rendant incompatible leur connexion avec le connecteur 5c comprenant le détrompeur 13. Ces moyens rendant incompatible la connexion d'éléments du kit avec le connecteur 5c comprenant le détrompeur 13 sont de manière préférentielle, des ailettes situées à l'extrémité desdits éléments du kit.

Le kit de préparation peut également comprendre des accessoires de prélèvement des solutions facilitant le remplissage des seringues de mélange 20 et 21.

Le kit de préparation K permet de mettre en oeuvre une méthode de préparation d'un mélange ou d'une émulsion destiné à l'injection chez un patient. Cette méthode comprend les étapes suivantes. Dans une première étape, deux seringues de mélange 20 et 21 sont montées sur deux connecteurs femelles correspondants 5a et 5b du robinet 1.

Dans une seconde étape, qui peut être réalisée simultanément à la première étape, la seringue d'injection 22 est montée sur le connecteur femelle 5c du robinet 1, comprenant le détrompeur 13.

De manière préférentielle, les seringues de mélange 20 et 21 contiennent comme contenu respectivement une solution aqueuse et une huile, préférentiellement une huile iodée. Ladite solution aqueuse comprend au moins un agent anti-cancéreux et optionnellement au moins un agent densifiant.

De façon avantageuse, l'agent anticancéreux que peut comprendre la solution aqueuse présente dans l'une des deux seringues de mélange, est choisi parmi les anthracyclines et plus préférentiellement parmi la doxorubicine, l'épirubicine, la némorubicine et l'idarubicine. Dans un mode de réalisation avantageux, la solution aqueuse peut ainsi comprendre en outre un agent densifiant, préférentiellement au moins un produit de contraste iodé non-ionique. Le produit iodé non ionique, utilisable en tant qu'agent densifiant, est, de manière préférée, choisi parmi l'iobitridol (Xenetix^{®}), l'iopamidol (Iopamiron^{®}, Isovue^{®}), l'iomeprol (Iomeron^{®}), l'ioversol (Optiray^{®}, Optiject^{®}), l'iohexol (Omnipaque^{®}), l'iopentol (Imagopaque^{®}), l'ioxitol (Oxilan^{®}), l'iopromide (Ultravist^{®}), le metrizamide (Amipaque^{®}), l'iosarcol (Melitrast^{®}), l'iotrolan (Isovist^{®}), l'iodixanol (Visipaque^{®}), l'iosiménol et l'iosimide (Univist^{®}) et un mélange de ceux-ci. L'iobitridol est le produit iodé non ionique préférentiel.

De façon avantageuse, l'huile iodée que peut contenir l'une des seringues de mélange comprend ou est constituée de dérivés d'acides gras iodés, préférentiellement d'esters éthyliques d'acides gras iodés, plus préférentiellement d'esters éthyliques d'acides gras iodés d'huile d'oeillette, d'huile d'olive, d'huile de graines de colza, d'huile d'arachide, d'huile de graines de soja ou d'huile de noix, encore plus préférentiellement d'esters éthyliques d'acides gras iodés d'huile d'œillette ou d'huile d'olive. Plus préférentiellement, cette huile iodée comprend ou est constituée d'esters éthyliques d'acides gras iodés d'huile d'œillette aussi appelée pavot noir ou *Papaver somniferum var. nigrum.*

Une troisième étape consiste à mélanger la solution aqueuse contenue dans la seringue de mélange 20 avec l'huile contenue dans la seringue de mélange 21 après avoir positionné le boisseau 3 de manière à mettre en communication les seringues de mélanges 20 et 21 par l'intermédiaire des conduits 2a et 2b et en effectuant un mouvement de va-et-vient des pistons des seringues de mélange 20 et 21 jusqu'à l'obtention d'un mélange ou d'une émulsion.

Ensuite, dans une quatrième étape, une partie ou la totalité du mélange ou de l'émulsion obtenu à l'étape de mélange est transférée dans la seringue d'injection 22, après avoir positionné le boisseau 3 de manière à mettre en communication la seringue de mélange 20, dans laquelle une partie ou la totalité du mélange ou de l'émulsion obtenu à l'étape de mélange a été transférée, avec la seringue d'injection 22 par l'intermédiaire du conduit 2a et du conduit 2c.

De manière préférentielle, la quatrième étape est réalisée dès que l'utilisateur du robinet estime visuellement que le mélange ou l'émulsion est homogène.

Le cathéter 23 est monté soit simultanément avec les seringues de mélange 20 et 21 et/ou avec la seringue d'injection 22, soit après réalisation de l'étape de mélange, soit après l'étape de transfert d'une partie du mélange ou de l'émulsion dans la seringue d'injection 22. De manière préférentielle, le cathéter 23 est monté sur le connecteur mâle 6, après réalisation de cette quatrième étape de transfert.

Le positionnement du boisseau 3 de manière à ce que la seringue d'injection 22 soit en communication avec le cathéter 23 puis l'actionnement du piston de la seringue d'injection 22 permettent ensuite l'injection directe du mélange ou de l'émulsion à un patient.

Une fois que les seringues de mélange 20 et 21, la seringue d'injection 22 et le cathéter 23 ont été connectés sur le robinet 1, une étape supplémentaire de mélange du mélange ou de l'émulsion obtenu à la troisième étape peut être réalisée, dans le cas où l'utilisateur estime visuellement que le mélange ou l'émulsion a déphasé. C'est un avantage important apporté par le robinet selon l'invention par rapport aux autres robinets de l'art antérieur qui imposent des déconnexions des seringues de mélange ou de la seringue d'injection, augmentant ainsi les risques d'entrée d'air dans le dispositif d'injection.

La mise en communication de la seringue de mélange 20 avec la seringue d'injection 22 ou de la seringue d'injection 22 avec le cathéter 23 se fait en positionnant de façon adéquate le canal de circulation de fluide 10 du boisseau 3 par un mouvement de rotation du levier 17. Le positionnement correct du boisseau 3 adapté à l'étape de préparation à effectuer est vérifié à l'aide des indicateurs fléchés 18. A titre d'exemple, sur la figure 11, le boisseau 3 est positionné de manière à mettre en communication fluidique les connecteurs 5a et 5b, comme l'indiquent les flèches 18, pour permettre le mélange des contenus des seringues de mélange 20 et 21.

Le détrompeur 13 permet d'éliminer le risque qu'une seringue de mélange 20 ou 21 ne soit connectée sur le connecteur 5c destiné à la seringue d'injection 22 et de erreurs soient commises dans la préparation du mélange qui doit être injecté au patient. Grâce au détrompeur 13, il n'y a qu'un connecteur femelle sur lequel la seringue d'injection 22 peut être montée, et les seringues de mélange 20 et 21 ne peuvent être montées que sur les connecteurs femelles restants, qui sont positionnés de manière contigüe. Cela assure la fiabilité du robinet médical 1.

Les caractéristiques des modes de réalisation et variantes décrits ci-dessus peuvent être combinées pour former de nouveaux modes de réalisation de l'invention.

## Revendications

1. Robinet médical (1) comprenant :
- un corps (2) muni de trois connecteurs femelles (5a, 5b, 5c) et d'un connecteur mâle (6), au moins un (5c) des connecteurs femelles (5a, 5b, 5c) étant adapté pour recevoir une seringue d'injection (22) tandis qu'au moins un des autres connecteurs femelles (5a, 5b) est adapté pour recevoir une seringue de mélange (20, 21) ayant un volume plus grand que celui de la seringue d'injection, et
- un boisseau (3) mobile monté dans le corps (2), muni d'un levier (17) de rotation et comprenant un canal de circulation de fluide (10),
**caractérisé en ce que** le connecteur femelle (5c) adapté pour recevoir la seringue d'injection (22) comprend un détrompeur (13) permettant d'empêcher le montage de la seringue de mélange (20, 21) sur ce connecteur femelle (5c), le détrompeur étant prévu pour empêcher que la seringue de mélange ne soit solidarisée mécaniquement et connectée fluidiquement à ce connecteur femelle (5c),
et **en ce que** le détrompeur (13) est formé de deux cornes (13a, 13b) prévues de part et d'autre du connecteur femelle (5c) adapté pour recevoir la seringue d'injection (22) et formant par rapport à ce connecteur femelle (5c) des espaces d'insertion (130, 131).

2. Robinet médical selon la revendication 1, **caractérisé en ce que** les cornes (13a, 13b) définissent une configuration mécanique qui est incompatible avec un moyen (20a) de la seringue de mélange (20, 21).

3. Robinet médical selon l'une des revendications précédentes, **caractérisé en ce que** le canal de circulation de fluide (10) met en communication fluidique uniquement deux connecteurs du robinet (1).

4. Robinet médical selon la revendication 3, **caractérisé en ce que** le canal de circulation de fluide (10) est en forme de L.

5. Robinet médical selon l'une des revendications précédentes, **caractérisé en ce que** deux connecteurs femelles contigus (5a, 5b) sont adaptés à une connexion avec des seringues de mélange (20, 21).

6. Robinet médical selon l'une des revendications précédentes, **caractérisé en ce qu'**il comprend au moins deux moyens de préhension (15) adaptés au positionnement d'un doigt.

7. Robinet médical selon la revendication 1, **caractérisé en ce que** le boisseau (3) comprend des indicateurs (18) permettant le repérage des connecteurs (5a, 5b, 5c, 6) qui sont mis en communication fluidique par le positionnement du boisseau (3).

8. Robinet médical selon la revendication 7, **caractérisé en ce que** le boisseau (3) comprend sur le dessous d'une partie supérieure (30) de ce boisseau (3), une rainure circulaire (31) interrompue par des nervures (32) alignées avec les indicateurs (18) et **en ce que** le corps (2) du robinet médical comprend sur sa partie supérieure un moyen (202) adapté pour coulisser dans la rainure circulaire (31) et être stoppé par les nervures (32), et empêchant une rotation du boisseau (3) qui mettrait en communication un connecteur femelle (5a, 5b) adapté à une connexion avec la seringue de mélange (20, 21) et le connecteur mâle (6).

9. Robinet médical selon la revendication 1, **caractérisé en ce que** le connecteur mâle (6) comprend une bague de verrouillage (8) mobile montée par clipsage sur l'extrémité distale dudit connecteur mâle (6).

10. Kit de préparation (K) d'un produit à injecter, préférentiellement un mélange ou une émulsion, **caractérisé en ce qu'**il comprend :
- un robinet médical (1) selon l'une des revendications précédentes,
- deux seringues de mélange (20, 21) adaptées pour être connectées à un premier et à un deuxième connecteur femelle (5a, 5b) du robinet (1), et
- une seringue d'injection (22) adaptée pour recueillir au moins une partie du produit obtenu par le mélange des contenus des seringues de mélange (20, 21) réalisé par un mouvement de va-et-vient des pistons desdites seringues de mélange (20, 21) et adaptée pour être connectée au connecteur femelle (5c) du robinet (1), muni du détrompeur (13).

11. Kit de préparation selon la revendication 10, **caractérisé en ce que** les seringues de mélange (20, 21) comprennent à leur extrémité des ailettes, formant un moyen rendant incompatible leur connexion avec le connecteur femelle (5c) muni du détrompeur (13).

12. Kit de préparation selon l'une des revendications 10 et 11, **caractérisé en ce que** la seringue d'injection (22) est une seringue qui comprend à son extrémité distale des godrons de préhension (220) qui ont un encombrement inférieur à la distance (D) entre des cornes (13a, 13b) du détrompeur (13).

13. Méthode de préparation d'un mélange ou d'une émulsion destiné à l'injection chez un patient, **caractérisée en ce qu'**elle comprend des étapes consistant à :
a) connecter deux seringues de mélange (20, 21) à deux connecteurs femelles correspondants (5a, 5b) d'un robinet médical (1) selon l'une des revendications 1 à 9 ;
b) connecter une seringue d'injection (22) au connecteur femelle (5c) du robinet médical (1), muni du détrompeur (13) ;
c) mélanger une solution aqueuse contenue dans l'une des seringues de mélange (20) avec une huile contenue dans l'autre seringue de mélange (21) après avoir positionné le boisseau (3) du robinet médical (1) de manière à mettre en communication fluidique les seringues de mélanges (20, 21) l'une avec l'autre et en effectuant un mouvement de va-et-vient des pistons de ces seringues de mélange (20, 21) jusqu'à l'obtention d'une émulsion ;
d) transférer une partie ou la totalité du mélange ou de l'émulsion obtenu à l'étape c) dans la seringue d'injection (22), après avoir positionné le boisseau (3) de manière à mettre en communication l'une des seringues de mélange (20, 21) avec la seringue d'injection (22).

14. Méthode de préparation selon la revendication 13, **caractérisée en ce qu'**elle comprend en outre une étape supplémentaire consistant à connecter un dispositif aval (23), de préférence un cathéter ou un micro-cathéter, au connecteur mâle (6) du robinet médical (1), cette étape étant soit concomitante à l'étape a), soit postérieure à l'étape b), soit concomitante à l'étape c), soit postérieure à l'étape d).

## Patentansprüche

1. Medizinisches Ventil (1), umfassend:
- einen Körper (2), der mit drei weiblichen Verbindern (5a, 5b, 5c) und einem männlichen Verbinder (6) ausgestattet ist, wobei mindestens einer (5c) der weiblichen Verbinder (5a, 5b, 5c) zur Aufnahme einer Injektionsspritze (22) geeignet ist, während mindestens einer der anderen weiblichen Verbinder (5a, 5b) zur Aufnahme einer Mischspritze (20, 21) mit einem größeren Volumen als dem der Injektionsspritze geeignet ist, und
- ein bewegliches Küken (3), das im Körper (2) montiert ist, mit einem Hebel (17) zur Drehung versehen ist und einen Kanal (10) zur Zirkulation von Fluid umfasst,
**dadurch gekennzeichnet, dass** der weibliche Verbinder (5c), der zur Aufnahme der Injektionsspritze (22) geeignet ist, eine Verwechselungssicherung (13) umfasst, die es ermöglicht, die Montage der Mischspritze (20, 21) an diesem weiblichen Verbinder (5c) zu verhindern, wobei die Verwechselungssicherung dazu vorgesehen ist, zu verhindern, dass die Mischspritze mechanisch fest mit diesem weiblichen Verbinder (5c) verbunden und fluidisch an diesen angeschlossen wird,
und dass die Verwechselungssicherung (13) aus zwei Hörnern (13a, 13b) gebildet ist, die auf beiden Seiten des weiblichen Verbinders (5c) vorgesehen sind, der dazu geeignet ist, die Injektionsspritze (22) aufzunehmen, und in Bezug auf diesen weiblichen Verbinder (5c) Einführräume (130, 131) bildet.

2. Medizinisches Ventil nach Anspruch 1, **dadurch gekennzeichnet, dass** die Hörner (13a, 13b) eine mechanische Konfiguration definieren, die mit einem Mittel (20a) der Mischspritze (20, 21) inkompatibel ist.

3. Medizinisches Ventil nach einem der vorangehenden Ansprüche, **dadurch gekennzeichnet, dass** der Fluidzirkulationskanal (10) nur zwei Verbinder des Ventils (1) in Fluidverbindung bringt.

4. Medizinisches Ventil nach Anspruch 3, **dadurch gekennzeichnet, dass** der Kanal zur Zirkulation von Fluid (10) L-förmig ist.

5. Medizinisches Ventil nach einem der vorangehenden Ansprüche, **dadurch gekennzeichnet, dass** zwei aneinandergrenzende weibliche Verbinder (5a, 5b) für eine Verbindung mit Mischspritzen (20, 21) geeignet sind.

6. Medizinisches Ventil nach einem der vorangehenden Ansprüche, **dadurch gekennzeichnet, dass** es mindestens zwei Greifmittel (15) umfasst, die zur Positionierung eines Fingers geeignet sind.

7. Medizinisches Ventil nach Anspruch 1, **dadurch gekennzeichnet, dass** das Küken (3) Indikatoren (18) umfasst, die die Ermittlung der durch die Positionierung des Kükens (3) in Fluidverbindung gebrachten Verbinder (5a, 5b, 5c, 6) ermöglichen.

8. Medizinisches Ventil nach Anspruch 7, **dadurch gekennzeichnet, dass** das Küken (3) auf der Unterseite eines oberen Teils (30) dieses Kükens (3) eine kreisförmige Nut (31) umfasst, die durch mit den Indikatoren (18) ausgerichtete Rippen (32) unterbrochen ist, und dass der Körper (2) des medizinischen Ventils an seinem oberen Teil ein Mittel (202) umfasst, das dazu geeignet ist, in der kreisförmigen Nut (31) zu gleiten und von den Rippen (32) gestoppt zu werden, und eine Drehung des Kükens (3) verhindert, die einen weiblichen Verbinder (5a, 5b), der für eine Verbindung mit der Mischspritze (20, 21) geeignet ist, und den männlichen Verbinder (6) in Verbindung bringen würde.

9. Medizinisches Ventil nach Anspruch 1, **dadurch gekennzeichnet, dass** der männliche Verbinder (6) einen beweglichen Verriegelungsring (8) umfasst, der durch Aufklipsen am distalen Ende des männlichen Verbinders (6) montiert ist.

10. Kit (K) zur Herstellung eines zu injizierenden Produkts, vorzugsweise einer Mischung oder einer Emulsion, **dadurch gekennzeichnet, dass** es Folgendes umfasst:
- ein medizinisches Ventil (1) nach einem der vorangehenden Ansprüche,
- zwei Mischspritzen (20, 21), die dazu geeignet sind, mit einem ersten bzw. einem zweiten weiblichen Verbinder (5a, 5b) des Ventils (1) verbunden zu sein, und
- eine Injektionsspritze (22), die dazu geeignet ist, mindestens einen Teil des Produkts aufzunehmen, das durch das Mischen der Inhalte der Mischspritzen (20, 21) erhalten wird, das durch eine Hin- und Herbewegung der Kolben der Mischspritzen (20, 21) durchgeführt wird, und die dazu geeignet ist, mit dem weiblichen Verbinder (5c) des Ventils (1), der mit der Verwechselungssicherung (13) versehen ist, verbunden zu sein.

11. Herstellungskit nach Anspruch 10, **dadurch gekennzeichnet, dass** die Mischspritzen (20, 21) an ihrem Ende Flügel umfassen, die ein Mittel bilden, das ihre Verbindung mit dem weiblichen Verbinder (5c), der mit der Verwechselungssicherung (13) versehen ist, inkompatibel macht.

12. Herstellungskit nach einem der Ansprüche 10 und 11, **dadurch gekennzeichnet, dass** es sich bei der Injektionsspritze (22) um eine Spritze handelt, die an ihrem distalen Ende Griffleisten (220) umfasst, deren Platzbedarf kleiner als der Abstand zwischen den Hörnern (13a, 13b) der Verwechselungssicherung (13) ist.

13. Verfahren zur Herstellung einer Mischung oder einer Emulsion zur Injektion in einen Patienten, **dadurch gekennzeichnet, dass** es folgende Schritte umfasst:
a) Verbinden von zwei Mischspritzen (20, 21) mit zwei entsprechenden weiblichen Verbindern (5a, 5b) eines medizinischen Ventils (1) nach einem der Ansprüche 1 bis 9;
b) Verbinden einer Injektionsspritze (22) mit dem weiblichen Verbinder (5c) des medizinischen Ventils (1), der mit der Verwechselungssicherung (13) ausgestattet ist;
c) Mischen einer wässrigen Lösung, die in einer der Mischspritzen (20) enthalten ist, mit einem Öl, das in der anderen Mischspritze (21) enthalten ist, nachdem das Küken (3) des medizinischen Ventils (1) so positioniert wurde, dass die Mischspritzen (20, 21) miteinander in Fluidverbindung stehen, und indem eine Hin- und Herbewegung der Kolben dieser Mischspritzen (20, 21) durchgeführt wird, bis eine Emulsion erhalten wird;
d) Überführen eines Teils oder der Gesamtheit der in Schritt c) erhaltenen Mischung bzw. Emulsion in die Injektionsspritze (22), nachdem das Küken (3) so positioniert wurde, dass eine der Mischspritzen (20, 21) mit der Injektionsspritze (22) in Verbindung steht.

14. Herstellungsverfahren nach Anspruch 13, **dadurch gekennzeichnet, dass** es ferner einen zusätzlichen Schritt umfasst, der darin besteht, eine stromabwärtige Vorrichtung (23), vorzugsweise einen Katheter oder einen Mikrokatheter, mit dem männlichen Verbinder (6) des medizinischen Ventils (1) zu verbinden, wobei dieser Schritt entweder gleichzeitig mit Schritt a) oder nach Schritt b) oder gleichzeitig mit Schritt c) oder nach Schritt d) erfolgt.

## Claims

1. Medical stopcock (1) comprising:
- a body (2) provided with three female connectors (5a, 5b, 5c) and a male connector (6), at least one (5c) of the female connectors (5a, 5b, 5c) being designed to receive an injection syringe (22), while at least one of the other female connectors (5a, 5b) is designed to receive a mixing syringe (20, 21) having a volume greater than that of the injection syringe, and
- a mobile plug (3) which is mounted in the body (2), is provided with a rotation lever (17) and comprises a fluid circulation channel (10),
**characterized in that** the female connector (5c) designed to receive the injection syringe (22) comprises a foolproofing device (13) to prevent the mixing syringe (20, 21) from being mounted on this female connector (5c), the foolproofing device being provided to prevent the mixing syringe from being mechanically secured and fluidically connected to this female connector (5c),
and **in that** the foolproofing device (13) is formed by two projections (13a, 13b) which are provided on each side of the female connector (5c) designed to receive the injection syringe (22) and which form insertion spaces (130, 131) with respect to this female connector (5c).

2. Medical stopcock according to Claim 1, **characterized in that** the projections (13a, 13b) define a mechanical configuration that is incompatible with a means (20a) of the mixing syringe (1).

3. Medical stopcock according to one of the preceding claims, **characterized in that** the fluid circulation channel (10) brings only two connectors of the stopcock (1) into fluidic communication.

4. Medical stopcock according to Claim 3, **characterized in that** the fluid circulation channel (10) is L-shaped.

5. Medical stopcock according to one of the preceding claims, **characterized in that** two contiguous female connectors (5a, 5b) are designed for a connection to mixing syringes (20, 21).

6. Medical stopcock according to one of the preceding claims, **characterized in that** it comprises at least two gripping means (15) designed for the placement of a finger.

7. Medical stopcock according to Claim 1, **characterized in that** the plug (3) comprises indicators (18) by which it is possible to identify the connectors (5a, 5b, 5c, 6) that are brought into fluidic communication by the positioning of the plug (3).

8. Medical stopcock according to Claim 7, **characterized in that** the plug (3) comprises, on the bottom of an upper part (30) of this plug (3), a circular groove (31) that is interrupted by ribs (32) aligned with the indicators (18), and **in that** the body (2) of the medical stopcock comprises, on its upper part, a means (202) designed to slide in the circular groove (31) and to be stopped by the ribs (32), preventing a rotation of the plug (3) that would establish a communication between a female connector (5a, 5b), designed for a connection to the mixing syringe (20, 21), and the male connector (6).

9. Medical stopcock according to Claim 1, **characterized in that** the male connector (6) comprises a movable locking ring (8) which is clipped onto the distal end of said male connector (6).

10. Preparation kit (K) for preparing a product to be injected, preferably a mixture or an emulsion, **characterized in that** it comprises:
- a medical stopcock (1) according to one of the preceding claims,
- two mixing syringes (20, 21) designed to be connected to a first and a second female connector (5a, 5b) of the stopcock (1), and
- an injection syringe (22) designed to collect at least some of the product obtained by the mixing of the contents of the mixing syringes (20, 21), effected by a reciprocating motion of the pistons of said mixing syringes (20, 21), and designed to be connected to the female connector (5c) of the stopcock (1), provided with the foolproofing device (13).

11. Preparation kit according to Claim 10, **characterized in that** the mixing syringes (20, 21) comprise fins at their end, forming a means that prohibits their connection to the female connector (5c) provided with the foolproofing device (13).

12. Preparation kit according to either of Claims 10 and 11, **characterized in that** the injection syringe (22) is a syringe which, at its distal end, comprises gripping beads (220) which have a size smaller than the distance (D) between projections (13a, 13b) of the foolproofing device (13).

13. Method for preparing a mixture or an emulsion intended for injection into a patient, **characterized in that** it comprises steps of:
a) connecting two mixing syringes (20, 21) to two corresponding female connectors (5a, 5b) of a medical stopcock (1) according to one of Claims 1 to 9;
b) connecting an injection syringe (22) to the female connector (5c) of the medical stopcock (1), provided with the foolproofing device (13);
c) mixing an aqueous solution, contained in one of the mixing syringes (20), with an oil contained in the other mixing syringe (21) after positioning the plug (3) of the medical stopcock (1) in such a way as to bring the mixing syringes (20, 21) into fluidic communication with each other, and effecting a reciprocating motion of the pistons of these mixing syringes (20, 21) until an emulsion is obtained;
d) transferring some or all of the mixture or emulsion obtained in step c) into the injection syringe (22) after positioning the plug (3) in such a way as to bring one of the mixing syringes (20, 21) into communication with the injection syringe (22).

14. Preparation method according to Claim 13, **characterized in that** it additionally comprises a supplementary step consisting in connecting a downstream device (23), preferably a catheter or a microcatheter, to the male connector (6) of the medical stopcock (1), this step being either concomitant with step a), or subsequent to step b), or concomitant with step c), or subsequent to step d).
